(19) **Europäisches Patentamt / European Patent Office / Office européen des brevets**

(11) **EP 2 000 934 A1**

(12) **EUROPEAN PATENT APPLICATION**

(43) Date of publication:
**10.12.2008 Bulletin 2008/50**

(51) Int Cl.:
**G06F 19/00** (2006.01)

(21) Application number: **07109798.4**

(22) Date of filing: **07.06.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Koninklijke Philips Electronics N.V.
5621 BA Eindhoven (NL)**

(72) Inventor: **The designation of the inventor has not yet been filed**

(74) Representative: **van Iersel, Hannie et al
Philips
Intellectual Property & Standards
P.O. Box 220
5600 AE Eindhoven (NL)**

(54) **A reputation system for providing a measure of reliability on health data**

(57) This invention relates to a system and a method for providing a measure of reliability on a first set of health data (102) on a patient (103) provided by a data provider. An assigner (105) is used for assigning the first set of health data or the data provider with at least one first rating element. A reputation-indicator (106) uses the at least one first rating element as input data in determining a first reputation measure indicating the reliability of the data provider. A comparer (107) then compares the determined first reputation measure with a pre-defined reputation threshold measure, the reputation threshold measure being a measure of a pre-set reliability level of the data provider.

Fig. 1

**Description**

FIELD OF THE INVENTION

The present invention relates to a reputation system and a method for providing a measure of reliability on a first set of health data on a patient.

BACKGROUND OF THE INVENTION

Electronic health records (EHR):

Nowadays, health data resides in the proprietary systems of healthcare providers. With the increased access to large networks, the possibility for a distributed system for health data arises, e.g. regional or national electronic health records (EHR) infrastructures. Healthcare professionals provide the information for these EHRs. The main objectives of EHRs are an increase of efficiency and quality of healthcare because of increased availability and reliability of health data. The Healthcare Information and Management Systems Society (HIMSS) provide a definition for an electronic health record *(T. Handler, R. Holtmeier, J. Metzger, M. Overhage, S. Taylor and C. Underwoord. HIMSS electronic health record definitional model version 1.1.2003)*. The Electronic Health Record (EHR) is a secure, real-time, point-of-care, patient centric information resource for clinicians. The EHR aids clinicians' decision making by providing access to patient health record information where and when they need it and by incorporating evidence-based decision support. The EHR automates and streamlines the clinician's workflow, closing loops in communication and response that result in delays or gaps in care. The EHR also supports the collection of data for uses other than direct clinical care, such as billing, quality management, outcomes reporting, resource planning, and public health disease surveillance and reporting. Currently, many countries are building and implementing electronic health records.

Personal health records (PHR):

A personal health record is a health record that is managed by the patient instead of the healthcare provider. Healthcare providers are not the only parties that can provide health data for the patient's well-being. Patients (but also people that are not ill, but are concerned about their health) may want to provide health information for their health records. Think for example of weight, heart rate and blood pressure information. Furthermore, as wellness providers such as fitness clubs and weight control clubs are professionalizing, they may want to use and provide relevant health data for the patient's health record. The health data supplied by the patient, wellness providers and healthcare providers is stored in the patient's PHR. Another reason for using a PHR is that healthcare providers are obliged to keep the patient records only for a certain period of time before they delete them. Patients that want to keep their health data after the data retention period can transfer them to their PHR. PHRs empower patients by providing them control over their health data. The patient may manage and share his health data in his PHR at his own discretion. After sharing, the health data in the PHR can be used by healthcare providers and wellness providers to improve the patient's health. Health data in a PHR is different from health data in an EHR. Health data in an EHR is always supplied (or at least reviewed) by a healthcare provider. Therefore, the health data that is in an EHR is considered accurate and trustworthy. In contrast, health data in a PHR can be supplied by healthcare providers, wellness providers and patients. Patients can supply their health data by adding the health data themselves, or by using a device that produces the health data and adds it to the PHR. Although the reliability of the health data supplied by patients and wellness providers cannot be guaranteed, this health data may be valuable for a healthcare provider. The health data supplied by the patient or by wellness providers can guide a healthcare provider in medical decision making, in making diagnoses, in deciding which medical tests and checks to do and in deciding whether to refer a patient to another healthcare provider. Considering the numerous PHR initiatives there is a trend in the USA that PHRs are more and more used *(http://www.e-health-insider.com/news/item.cfm?ID=2520)*.

Parties and the reliability of their health data:

Healthcare providers are persons with a background in medicine, therefore health data originating from a healthcare provider can be expected to have a certain level of accuracy. Moreover, healthcare providers are certified as such, and are listed in national registers. Therefore, healthcare providers are expected to produce highly reliable health data for both EHRs and PHRs. Health data supplied by patients is of varying reliability because patients have varying medical knowledge. Chronically ill patients taking measurements every day are likely to provide very accurate health data. However, many patients do not exactly know how to take measurements. Elderly people may have difficulties taking measurements. In general, the reliability of patient-supplied health data may vary from useless to a level comparable to health data supplied by a healthcare provider. As with patient-supplied health data, health data supplied by wellness providers can also be of varying quality. Wellness providers that supply health data are often specialized in their field of expertise. Therefore, the reliability of the supplied health data is probably not very bad. The reliability of this health data depends on the quality of the devices, the level of training of the employees of the wellness provider and the level of training the wellness provider gives to their consumers.

Therefore, it is very difficult to evaluate the reliability of existing health data.

BRIEF DESCRIPTION OF THE INVENTION

The object of the present invention is to provide a system and a method for determining the reliability of health data on a patient.

According to one aspect the present invention relates to a reputation system for providing a measure of reliability on a first set of health data on a patient provided by a data provider, the system comprising:

a) an assigner for assigning the first set of health data or the data provider with at least one first rating element,
b) a reputation-indicator for using at least one first rating element as input data in determining a first reputation measure indicating the reliability of the data provider, and
c) a comparer for comparing the determined first reputation measure with a pre-defined reputation threshold measure, the reputation threshold measure being a measure of a pre-set reliability level of the data provider.

Accordingly, by evaluating the data provider in this way, it is possible to evaluate the reliability of the health data. This gives e.g. healthcare providers the advantage of making an informed decision on the reliability of health data based on the reputation of the data provider with almost no overhead for healthcare providers, wellness providers and patients.

In one embodiment, the assigner comprises a rule engine adapted to determine ratings for the data provider associated with certificates of the data provider.

Therefore, a relevant parameter or a measure is provided about the data provider, namely if he/she is qualified or not in collecting health data.

In one embodiment, the assigner comprises an aggregation engine adapted to determine ratings for the health data based on comparing two or more health data sets on the same patient provided by different data providers.

Accordingly, if e.g. two data sets are very similar a good indicator is provided indicating that the data provider is reliable.

In one embodiment, the aggregation engine determines the ratings for the health data by calculating the consistency between two or more health data sets.

In one embodiment, the assigner comprises a healthcare provider or a wellness provider which assigns by a manual operation the health data with the at least one rating element.

Accordingly, a manual operation is also possible to assign the health data with one or more rating elements. As an example, the healthcare provider can e.g. be a doctor, dentists, surgeons or any kind of expert who is present when the patient creates the health data. After observing the patient, the healthcare provider can manually assign the data with a reliable rating element.

In one embodiment, the rating means comprises a metadata source for incorporating metadata to the health data, the metadata being implemented as additional data source in assigning the health data with at least one rating element.

Accordingly, such metadata can e.g. include whether the patient was steady during the measurement, whether the measurement condition was correct, the type of the measurement device and thus the accuracy of the measurement device etc. All these additional information can be highly relevant when assigning the health data with one or more rating element.

In one embodiment, the data provider is a patient or a wellness provider. In another embodiment, the data provider is a healthcare provider.

In one embodiment, the first set of health data as well as the accompanying metadata is obtained from an external database or system over a communication channel.

In one embodiment, the first reputation measure related to health data is sent over a communication channel to a remote system.

In one embodiment, the rating relating to a first set of health data is sent over a communication channel to a remote system

According to another aspect, the present invention relates to a method of providing a measure of reliability on a first set of health data on a patient provided by a data provider, comprising:

a) assigning the first set health data or the data provider with at least one rating element,

b) using the assigned rating element as input data in determining a first reputation measure indicating the reliability of the data provider, and

c) comparing the determined first reputation measure with a pre-defined reputation threshold measure, the reputation threshold measure being a measure of a pre-set reliability level of the data provider.

According to yet another aspect, the present invention relates to a computer program product for instructing a processing unit to execute the method step of the method when the product is run on a computer.

The aspects of the present invention may each be combined with any of the other aspects. These and other aspects of the invention will be apparent from and elucidated with reference to the embodiments described hereinafter.

BRIEF DESCRIPTION OF THE DRAWINGS

Embodiments of the invention will be described, by way of example only, with reference to the drawings, in which

Figure 1 shows a reputation system according to the present invention for providing a measure of reliability of a first set of health data on a patient,

Figure 2 is a block diagram of one embodiment of a reputation system according to the present invention,

Figure 3 depicts graphically one embodiment of an interaction between a healthcare provider and the PHR system, and

Figure 4 shows a flowchart of a method according to the present invention for providing a measure of reliability of health data on a patient provided by a data provider.

DESCRIPTION OF EMBODIMENTS

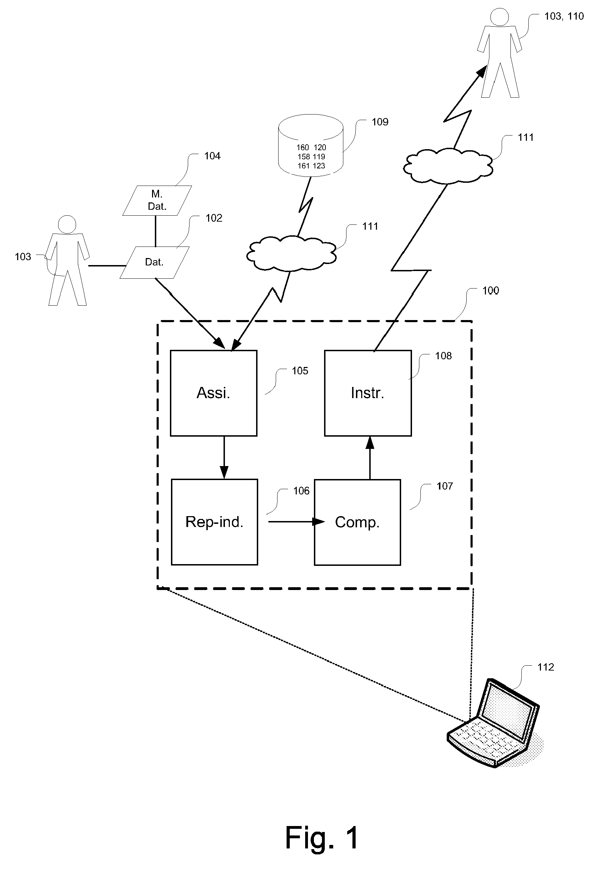

Figure 1 shows a reputation system 100 according to the present invention for providing a measure of reliability on a first set of health data 102 on a patient 103 provided by a data provider. The data provider can be the person that created the health data on the patient, e.g. the patient himself, a healthcare provider (e.g. a doctor, dentist or any person qualified to perform such measurements), a wellness provider (e.g. a person of a fitness center), or a computer or similar means that is pre-programmed to perform such measurements. The system comprises an assigner 105, a reputation-indicator 106 and a comparer 107.

The role of the assigner 105 is to assign ratings to the health data 102 or the data provider 103. In one embodiment the assigner 105 is a rule engine that computes rule ratings by relying on certificates of the data provider, e.g. the patient if he/she created the health data himself, or the wellness provider if it was the wellness provider that created the health data on the patient, or the healthcare provider if it is the healthcare provider that created the health data on the patient. Accordingly, the reliability of the data provider is reflected in the rule rating. The rule engine may implement different methods to find the rule ratings. As an example, the rule engine may be adapted to use a predefined mapping to find the rule ratings associated with a certificate. The certificates may e.g. be university diplomas, school or accreditation organization, tests, different types of courses, e.g. online courses etc. As an example, if the health data are created by the patient and the certificate indicates that he/she is very qualified in performing such a measurement, e.g. has participated in a number of courses related to how to create such health data, the rule rating would be evaluated as being high. Accordingly, the rule rating element reflects the "educational level" of the one that created the health data on the patient and is thus a good indicator for the "reliability" of the health data.

In one embodiment, the assigner 105 is an aggregation engine adapted to determine aggregation ratings based on comparing health data created by different data providers, preferably with a small time difference because the information can change with time. As an example, if the data providers is a doctor A and a patient B that do the same measurement on the patient and these measurements are similar, the aggregation ratings will be high, whereas an inconsistency in the measurements would result in lower aggregation ratings. Accordingly, the aggregation rating reflects a statistical reliability of the measured data.

In one embodiment, the assigner 105 is a healthcare provider, e.g. a doctor, which assigns health data that he/she receives with rating(s) after checking the data. This would typically be done based on being present when the data provider creates the health data, repeating creation of the health data etc. The rating assigned by the healthcare provider is referred to as local rating. As an example a doctor might assign blood pressure values measured by a patient over one week with ratings by assigning either the complete data set with a single rating element, or by assigning each data element with a rating element. This will be discussed in more details later.

In case the ratings provided for a data provider are provided by other healthcare providers, the ratings are referred to as global ratings.

Accordingly, the rating elements define the reliability parameter relating to the reliability of the measurement performed on the patient. As mentioned previously, if the rule rating is high the qualification of the data provider that created the health data is high, the local rating is e.g. given by a doctor assigning e.g. each respective data element with a rating, the global rating is when e.g. a second healthcare provider uses the local rating assigned by another healthcare provider, the aggregation rating relies on statistical procedure by comparing two or more measurements together and based thereon assigning the health data with an aggregation rating element. These rating elements serve as input for subsequent step performed by the reputation-indicator 106.

The role of the reputation-indicator 106 is to use the assigned rating elements as input data or input parameters in determining a first reputation measure for the data provider of the first set of health data. This first reputation measure determines the reputation of the data provider and thus reflects the reliability of the first set of data, i.e. whether they are trustworthy or not. A typical criterion is that they are accurate; the reliability is high, etc. The reputation-indicator 106 will be discussed in more details later.

The comparer 107 compares the first reputation measure with a threshold measure which is a measure of a pre-set reliability level of the data provider. The comparer can be e.g. a doctor, which is well aware of a reference level (a threshold measure) for evaluating whether the first reputation measure is sufficiently high and thus whether the reliability of the health data is sufficiently high. The comparer 107 can also be e.g. a processor which is pre-programmed to automatically compare the first reputation measure with a pre-set threshold measure.

In one embodiment, the reputation system 100 further comprises an instructor 108 to instruct, in case the determined reputation measure is below the reputation threshold measure, whether recreation of the health data is necessary or not based on whether the result from the comparer 107. If the determined reputation measure is below the reputation threshold measure at least a second set of health data can be created by the same data provider or a new data provider or the data could be discarded. Subsequently, at least one second rating elements are assigned to the second set of health data, a second reputation measure is determined based on the at least one second ratings elements and finally the second reputation measure is compared with the reputation threshold measure. This is repeated until the subsequent determined reputation measure reaches or exceeds the reputation threshold measure.

As depicted in Fig. 1, the health data may be personal health records (PHR) or electronic health records (EHR) obtained from an external database 109 over e.g. a wireless communication channel 111 such as the internet. Thus, a doctor can be provided with health data on the patient and check whether the health data are of high reliability or not.

The result of the instructor 108 could also be forwarded to an external agent 103, 110, e.g. a doctor 110 or the patient 103 over a wireless communication channel 111. If the response indicates that the reliability of the first set of health was not high enough, a data provider might be requested to repeat the measurements.

In one embodiment, the health data 102 are associated with metadata 104 for indicating how the health data creation process on the patient was performed, e.g. whether the blood pressure meter was placed at correct height during the measurement, or whether the patient was steady during the measurement etc. Accordingly, the metadata may reflect the context or circumstances of the measurement. This information may be very valuable for e.g. the doctor when assigning the local ratings simply by looking at the metadata. Such metadata are typically provided by the device that issues and attaches the metadata to the health data.

The above mentioned system could be integrated into e.g. a computer device 112, e.g. a PC computer, PDA or similar means comprising a processor for performing the above mentioned steps.

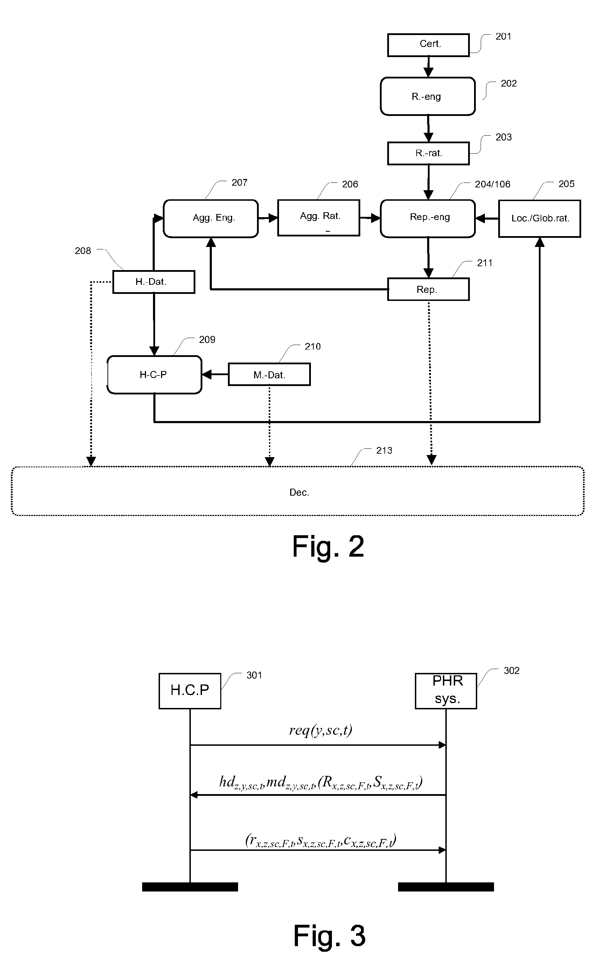

Figure 2 is a block diagram of one embodiment of a reputation system 100 according to the present invention, where the assigners 105 consist of rule engine 202, aggregation engine 207, and a healthcare provider 209.

The rule engine 202 can be a computer 202 or similar means comprising a processor where the computation relies on available certificates 201, e.g. pre-stored in a digital database. This would typically be digital information identifying certificates associated with the data provider. If e.g. no certificate is found about the data provider, the assigned rule elements 203 might be considered to be low (or zero), whereas if highly recognized certificate would be found for the health data provider (e.g. a doctor a the health data provider) the rule elements would be high.

The aggregation engine 207 typically performs a statistical evaluation or calculation on two or more sets of health data 208 on a patient and based thereon issues aggregation rating elements 206. Such a statistical evaluation could be performed by a computer or similar means.

The healthcare provider 209 assigns the health data 208 with local or global rating elements 205. As discussed previously, local rating is where e.g. a healthcare provider assigns the health data with rating element(s). As an example, patient A takes measurement and doctor B gives rating. If doctor B would calculate reputation of A this rating would be a local rating.

The global rating is where another healthcare provider uses a local rating to determine a first reputation measure. As an example, doctor C can use the rating provided by doctor B to calculate the reputation of patient A. In order to use the rating of doctor B, doctor C calculates a global rating (based on the rating given by doctor B).

To summarize, if doctor B gives a rating, this rating is a local rating for doctor B, and a global rating for doctor C.

As mentioned previously, in some cases the health data 208 has associated metadata 210 that provide additional information about e.g. the measurement procedure, or the type of the measurement device being used etc. These data can be highly relevant when assigning the health data with local rating elements. As an example, information about the model, e.g. the type/brand, of the measurement device that the patient used can be a relevant factor in evaluating how reliable the measurements are. Also, the additional information indicating that e.g. the relative position of the arm was correct when the blood pressure was measured could also be considered as highly relevant information.

These assigned rating elements are used as input data for calculating the reputation-measure 106/204, which determines the reputation measure 211 of the data provider, e.g. the healthcare provider, wellness provider, the patient. The higher the reputation measure is the higher is the reliability of the health data, and vice verse, the lower the reputation measure is the lower is the reliability of the health data.

If the reputation measure is below a pre-defined threshold reputation measure the health data should preferably be recreated, e.g. different measuring device, or a different data provider creating the health data.

When the reputation measure 211 is equal or larger than the threshold measure, a decision is issued 213 indicating whether the data provider, and thus the health data, may be considered to be reliable.

Begin of an embodiment

In this example, the rating element is a tuple (r,s,c) where r is the positive fraction of the rating, s is the negative fraction of the rating and c is the certainty of the rating. r, s and c are all real numbers between 0 and 1 and r and s satisfy the condition r+s=1. A reputation or reputation part is an aggregation of ratings. A reputation (part) is a tuple (R, S) where R is the combination of the positive fractions r of the ratings and S is the combination of the negative fractions s of the ratings. This model is an extension of the model for ratings and reputations introduced by Jøsang and Ismail *(A. Josang and R. Ismail, The Beta Reputation System, In Proc. 15th Bled Conf. Electronic Commerce, 2002)* hereby incorporated by reference.

The four different kinds of ratings (local, global, aggregation and rule ratings) can be divided into two categories: ratings for health data and rule ratings. Ratings from both categories need to be combined in different ways. In the next sections the different ways of combining the ratings of the different categories are discussed.

Combining ratings on health data:

This reputation part is based on ratings on health data (local, global and aggregation ratings). The positive fraction R of the reputation part is calculated by summing all positive fractions r of the local ratings. The positive fractions r of the ratings are scaled by several factors:

- The certainty c: a rating with a high certainty should be given more weight than a rating with a low certainty.
- The first forgetting function: a function that gives more weight to more recent ratings. Users may start behaving better (or worse) over time. Therefore, recent ratings should be given more weight than older ratings. The last rating should be given the highest weight, the one before slightly less weight, etc.
- The second forgetting function: a function that gives more weight if the health data was created closer in time. As the time between the creation of the health data and the calculation of the reputation part increases, the rating should be given less weight. After all, if a user performed good (or bad) a very long time ago, there is no guarantee that he will do so now.
- The similarity in scope function: if not enough ratings are available for calculating the reputation, ratings for other scopes can be used. A scope is a pair (m,d) where m is the measurement kind (e.g. blood pressure) and d is the device (e.g. Philips HF305 blood pressure meter) that is used. For the ratings to be useful, the scope for which the reputation is calculated has to be correlated to the scope of the ratings. The scope function is a function that gives more weight if the scopes are close to each other. This function has initial values for every pair of scopes and is updated dynamically.

A reputation is the subjective judgment of a user x about a user y. A reputation is always calculated for a scope sc, a trusttype tt (either functional or recommendation) and for a time t. The positive fraction RH and the negative fraction SH of the reputation part are calculated as follows:

$$RH_{x,y,sc,tt,t} = \sum_{j=1}^{|SC|} SS(sc, sc_j) \cdot \sum_{i=1}^{|H_j|} g(t_i, t) \cdot f(i, |H_j|) \cdot c^i_{x,y,sc_j,tt,t_i} \cdot r^i_{x,y,sc_j,tt,t_i}$$

$$SH_{x,y,sc,tt,t} = \sum_{j=1}^{|SC|} SS(sc,sc_j) \cdot \sum_{i=1}^{|H_j|} g(t_i,t) \cdot f(i,|H_j|) \cdot c^i_{x,y,sc_j,tt,t_i} \cdot s^i_{x,y,sc_j,tt,t_i}$$

With:

| | |
|---|---|
| SC | The set of scopes. |
| SS(sc,scj) | A function returning the similarity between scopes sc and scj. |
| $H_j$ | The set of local, global and aggregation ratings $(r_{x,y,scj,tt,t_i}$, $S_{x,y,scj,tt,t_i}$, $C_{x,y,scj,tt,t_i})$ for scope j, ordered ascendingly by time of creation of the health data. Only ratings with time $t_i < t$ are present in the set. |
| $g(t_i,t)$ | The second forgetting function, giving higher values when $t_i$ is closer to t. |
| $f(i,|H_j|)$ | The first forgetting function, giving higher values when i is closer to $|H_j|$. |
| $r_{x,y,sc,tt,t}$ | The positive fraction of the rating of x about y for scope sc, trust type tt and at time t. |
| $s_{x,y,sc,tt,t}$ | The negative fraction of the rating of x about y for scope sc, trust type tt and at time t. |
| $C_{x,y,sc,tt,t}$ | The certainty of the rating of x about y for scope sc, trusttype tt and at time t. |

Combining rule ratings:

This reputation part is based on rule ratings. The positive fraction RR and the negative fraction SR of the reputation part are calculated as follows:

$$RR_{x,y,sc,tt,t} = \sum_{i=1}^{|R|} c^i_{y,sc,tt,t_i} \cdot r^i_{y,sc,tt,t_i}$$

$$SR_{x,y,sc,tt,t} = \sum_{i=1}^{|R|} c^i_{y,sc,tt,t_i} \cdot s^i_{y,sc,tt,t_i}$$

With:

| | |
|---|---|
| R | The set of rule ratings $(r_{y,sc,tt,t}$, $S_{y,sc,tt,t}$, $C_{y,sc,tt,t})$ determined by the rule engine based on the possession of certificates by user y. Only ratings with time $t_i < t$ are present in the set. |
| $r_{y,sc,tt,t}$ | The positive fraction of the rating of x about y for scope sc, trust type tt and at time t. This value is determined by the rule engine based on possession of a certificate by user y. |
| $S_{y,sc,tt,t}$ | The negative fraction of the rating of x about y for scope sc, trust type tt and at time t. This value is determined by the rule engine based on possession of a certificate by user y. |
| $C_{y,sc,tt,t}$ | The certainty of the rating of x about y for scope sc, trust type tt and at time t. This value is determined by the rule engine based on possession of a certificate by user y. |

Combining rating on health data and rule ratings:

The two reputation parts (one on health data ratings and one on rule ratings) can be combined to obtain the reputation. The positive fraction R and the negative fraction S of the reputation are calculated as follows:

$$R_{x,y,sc,tt,t} = RH_{x,y,sc,tt,t} + \omega \cdot RR_{x,y,sc,tt,t}$$

$$S_{x,y,sc,tt,t} = SH_{x,y,sc,tt,t} + \omega \cdot SR_{x,y,sc,tt,t}$$

where ω is the weight given to the reputation part based on rule ratings (ω ≥ 0).

Calculation of the ratings:

Local ratings: A local rating is a rating provided by a healthcare provider x after he checked the reliability of the health data that y supplied.

*Global ratings:* To gain a broader view about a user y, a user x can ask other users z about their ratings for y. Ratings from z should be discounted (i.e. given less weight) because a user's own ratings are always more reliable than another user's ratings. The ratings of other users are discounted by the recommendation reputation of the user z (the supplier the rating). The recommendation reputation of a user z is a pair ($R_{x,z,sc,R,t}$, $S_{x,z,sc,R,t}$) is calculated similarly to the functional reputation. However, for the recommendation only rule ratings and no ratings for health data are used.

Clearly, ratings from users with high recommendation reputation should be given more weight than users with low recommendation reputation. Therefore a rating of a user z can be easily discounted by his recommendation reputation by discounting the certainty of the rating. The discounted rating can then be calculated as follows:

$$r_{x,y,sc,tt,t} = r_{z,y,sc,tt,t}$$

$$s_{x,y,sc,tt,t} = s_{z,y,sc,tt,t}$$

$$c_{x,y,sc,tt,t} = \frac{R_{x,z,sc,R,t}}{R_{x,z,sc,R,t} + S_{x,z,sc,R,t} + 2} \cdot c_{z,y,sc,tt,t}$$

*Aggregation ratings:* The aggregation engine provides aggregation ratings that can be used for reputation computation by the reputation engine. An aggregation rating is computed by comparing measurements from different sources with a small time difference. If two users do the same measurement on the same person and these measurements are similar, then the reputation of both users can be increased. If two users do the same measurement on the same person and the measurements are not similar, then the reputation of both users must be decreased (In case of two users with similar reputations, this makes perfect sense. In case of a healthcare provider repeating a measurement of a patient, it is not necessary to change the reputation of the healthcare provider. In practice, the reputation of users with a very high reputation (e.g. healthcare providers) is only very slightly changed. Suppose $hd_{y,z,(m,d),t}$ is a measurement of user y on user z at time t. The measurement is of kind m and is taken with device d. D is a set of measurements $hd_{yi,z(m,di)ti}$ of the same kind and on the same person. D is chosen such that the measurements in D are close enough in time to infer information about the correctness of one of the measurement from the other(s). An aggregation rating can be calculated for $hd_{y,z,(m,d),t}$ as follows:

$$r_{x,y,(m,d),F,t} = SH(hd_{y,z,(m,d),t}, D, m)$$

$$s_{x,y,(m,d),F,t} = 1 - SH(hd_{y,z,(m,d),t}, D, m)$$

$$c_{x,y,(m,d),F,t} = \frac{\sum_{i=1}^{|D|} ST(t,t_i,m) \cdot R_{x,y_i,(m,d_i),F,t}}{\sum_{i=1}^{|D|} ST(t,t_i,m) \cdot (R_{x,y_i,(m,d_i),F,t_i} + S_{x,y_i,(m,d_i),F,t_i}) + 2}$$

where $SH(hd_{y,z,(m,d),t}, ,D,m)$ is the function that compares the measurement $hd_{y,z,(m,d),t}$ to the measurements in the set D. As part of calculating SH, first the most probable value for $hd_{y,z(m,d),t}$ based on the measurements in the set D is calculated. Second, the similarity between $hd_{y,z,(m,d),t}$ and this most probable value is computed.

The most probable value is a weighted average of the measurements of the same kind on the same patient:

$$mpv(\mathrm{hd}_{y,z,(m,d),t}, D) = \frac{\sum_{i=1}^{|D \cup \{hd_{y,z,(m,d),t}\}|} ST(t,t_i,m) \cdot hd_{y_i,z(m,d_i),t_i}}{\sum_{i=1}^{|D \cup \{hd_{y,z,(m,d),t}\}|} ST(t,t_i,m)}$$

The weights of the equation are the similarities in time between the measurements. The similarity in time $ST(t,t_i,m)$ is calculated as follows:

$$ST(t,t_i,m) = e^{\frac{-(t-t_i)^2}{2 \cdot \sigma_{time,m}^2}}$$

where $\sigma_{time,m}$ is the standard deviation for time belonging to measurement kind m.

The similarity of the measurement $hd_{y,z,(m,d),t}$ is then calculated by:

$$SH(\mathrm{hd}_{y,z,(m,d),t}, D, m) = e^{\frac{-(\mathrm{hd}_{y,z,(m,d),t} - mpv(\mathrm{hd}_{y,z,(m,d),t},D))^2}{2 \cdot \sigma_{hd,m}^2}}$$

where $\sigma_{hd,m}$ is the standard deviation for health data belonging to measurement kind m.

*Rule ratings:* The rule engine computes rule ratings that can be used by the reputation engine. The computation relies on available certificates. Certificates may be diplomas from a university, school or accreditation organization. Another possibility for obtaining a certificate is by following an online course and passing a test. A certificate is represented as a tuple (x, p, t) stating property p about user x, where p can be any property leading to a rule rating (e.g. 'completed medical school' or 'successfully completed online tutorial for measuring blood pressure') and t is the time of creation of the certificate.

The rule engine maps certificates to rule ratings ($r_{x,sc,tt,t}$, $s_{x,sc,tt,t}$, $c_{x,sc,tt,t}$) using a predefined mapping. Every time a new type of certificate is accepted, or a new scope is introduced, the mapping has to be updated. The mapping can be represented as a lookup table.

Interactions with the PHR system:

The interactions between the patient and the PHR system as well as between the wellness provider and the PHR system have changed in such a way that the health data that is sent to the PHR system by these suppliers or data providers can be accompanied by metadata. This metadata can be used by the PHR system and the healthcare provider to calculate a rating. For the healthcare provider, the interaction with the PHR system has changed such that they can obtain reputations and supply ratings (A situation where the wellness provider (and even the patient) would also obtain the reputation of the data provider of the health data will also be needed. After all, a reputation is (and should be) public information. In this situation, the interactions between the wellness provider (and patient) and PHR system are similar to the one in figure 3). Instead of obtaining health data from the PHR system, the healthcare provider also obtains metadata on this health data and the reputation of the data provider of the health data at the time of creation of the health data. After obtaining the health data, the healthcare provider can choose to supply a rating for the health data. The other option is that the healthcare provider repeats a measurement and adds the measurement to the PHR of the user. The reputation system then automatically calculates an aggregation rating.

One embodiment of the interaction between a healthcare provider 301 and the PHR system 302 is depicted in figure 3, and includes the following steps:

Healthcare provider 301 x requests health data on patient y for scope sc created at time t.

The PHR system 302 verifies the identity of x and if x is has sufficient access rights, the PHR system sends the health data to x. If any metadata provided by the device is available, the PHR system also sends this to x. The health data and metadata are accompanied by the reputation of the data provider of the data at time of creation of the data.

If no rating has been provided for this health data, x sends his rating for the health data to the PHR system. This step is optional.

End the embodiment.

Begin example 1:

Blood pressure (part 1):

"Lately, Alice has not been feeling well. She sometimes has a blurred vision and she regularly has headaches. Alice decides to pay her general practitioner, Dr. Bob, a visit. Bob measures Alice's blood pressure and finds her blood pressure to be rather high (160/100 mmHg). High blood pressure significantly increases the risk of heart failure and the risk of stroke. Therefore, Bob decides that from now on, Alice has to measure her blood pressure every day. Alice decides she will measure her blood pressure using a sphygmomanometer. As blood pressure changes during the day, Alice always measures her blood pressure at the end of her working day. In the first week, Alice measures the blood pressure values depicted in table 1.

**Table 1: Blood pressure values of the first week**

| Day | Systolic BP | Diastolic BP |
|---|---|---|
| Monday | 170 mmHg | 110 mmHg |
| Tuesday | 165 mmHg | 105 mmHg |
| Wednesday | 140 mmHg | 90 mmHg |
| Thursday | 155 mmHg | 100 mmHg |
| Friday | 160 mmHg | 100 mmHg |

Bob is not quite sure about the measurements Alice provides. He expresses this by providing average ratings with very high uncertainty."

Bob provides the following ratings:

$$(r_{B,A,(bp,sphyg),101}, s_{B,A,(bp,sphyg),101}, c_{B,A,(bp,sphyg),101}) = (0.5, 0.5, 0.2)$$

$$(r_{B,A,(bp,sphyg),102}, s_{B,A,(bp,sphyg),102}, c_{B,A,(bp,sphyg),102}) = (0.5, 0.5, 0.2)$$

$$(r_{B,A,(bp,sphyg),103}, s_{B,A,(bp,sphyg),103}, c_{B,A,(bp,sphyg),103}) = (0.5, 0.5, 0.2)$$

$$(r_{B,A,(bp,sphyg),104}, s_{B,A,(bp,sphyg),104}, c_{B,A,(bp,sphyg),104}) = (0.5, 0.5, 0.2)$$

$$(r_{B,A,(bp,sphyg),105}, s_{B,A,(bp,sphyg),105}, c_{B,A,(bp,sphyg),105}) = (0.5, 0.5, 0.2)$$

"Because the ratings vary a lot, Bob asks Dr. Charlie to check on Monday whether Alice measures her blood pressure in a proper way. As Charlie is Alice's company doctor, he has completed medical school and therefore he can provide recommendations to Bob. Unfortunately, Charlie observes that Alice measures her blood pressure quite clumsily and that she has quite some difficulties handling the sphygmomanometer. Therefore, Charlie provides a bad rating for Alice's measurement."

Charlie's rating for Alice is represented by:

$$(r_{C,A,(bp,sphyg),F,108}, \ s_{C,A,(bp,sphyg),F,108}, \ c_{C,A,(bp,sphyg),F,108}) = (0.1, 0.9, 1)$$

Charlie's recommendation reputation is represented by:

$$R_{B,C,(bp,sphyg),R,108} = 11.25 \qquad S_{B,C,(bp,sphyg),R,108} = 1.25$$

The global rating of Bob for Alice through Charlie is then calculated by:

$$r_{B,A,(bp,sphyg),F,108} = 0.1$$

$$s_{B,A,(bp,sphyg),F,108} = 0.9$$

$$c_{B,A,(bp,sphyg),F,108} = \frac{11.25}{11.25+1.25+2} \cdot 1 = 0.7759$$

"Providing highly reliable measurements is in the interest of both Alice and Bob. Therefore, Bob recommends using a Braun BP3550 blood pressure monitor with advanced positioning sensors. For measuring blood pressure, it is important to position the blood pressure meter at heart level. The health data provided by the BP3550 is accompanied by metadata. This metadata contains information on whether the arm was positioned at the right level. Therefore, Bob can make a more informed decision on the reliability of the health data. Additionally, Alice takes an online tutorial on how to measure her blood pressure using the Braun BP3550. Therefore, her reputation for taking blood pressure measurements using the BP3550 is increased."

The rule engine has calculated the following rule rating for Alice:

$$(r_{A,(bp,BP3550),F,108}, \ s_{A,(bp,BP3550),F,108}, \ c_{A,(bp,BP3550),F,108}) = (0.7, 0.3, 0.1)$$

"The blood pressure values for the rest of the second week are depicted in table 2."

**Table 2: Blood pressure values for the second week**

| Day | Systolic BP | Diastolic BP |
|---|---|---|
| Tuesday | 162 mmHg | 101 mmHg |
| Wednesday | 158 mmHg | 102 mmHg |
| Thursday | 155 mmHg | 95 mmHg |
| Friday | 160 mmHg | 101 mmHg |

Bob provides the following ratings:

$$(r_{B,A,(bp,BP3550),109}, \; s_{B,A,(bp,BP3550),109}, \; c_{B,A,(bp,BP3550),109}) = (0.8, \, 0.2, \, 0.6)$$

$$(r_{B,A,(bp,BP3550),110}, \; s_{B,A,(bp,BP3550),110}, \; c_{B,A,(bp,BP3550),110}) = (0.8, \, 0.2, \, 0.6)$$

$$(r_{B,A,(bp,BP3550),111}, \; s_{B,A,(bp,BP3550),111}, \; c_{B,A,(bp,BP3550),111}) = (0.8, \, 0.2, \, 0.6)$$

$$(r_{B,A,(bp,BP3550),112}, \; s_{B,A,(bp,BP3550),112}, \; c_{B,A,(bp,BP3550),112}) = (0.8, \, 0.2, \, 0.6)$$

"As the blood pressure values remain to be too high, Alice pays another visit to Bob. Bob asks Alice to do a measurement using her blood pressure meter. After Alice has done her measurement, Bob immediately repeats Alice's measurement using his own equipment. This way, Bob can be sure that Alice's blood pressure has not changed between the two measurements. The system automatically observes that the measurements of Alice and Bob are very close and as Bob is a doctor, Alice's reputation is increased."

Alice measures a blood pressure value of 179 and Bob measures a blood pressure value of 180. Therefore, the aggregation rating is high. Because Bob measures the blood pressure within a minute, the certainty of the rating is also high. The aggregation engine calculates the following aggregation rating, based on Alice's and Bob's measurement:

$$(r_{B,A,(bp,BP3550),F,122}, \; s_{B,A,(bp,BP3550),F,122}, \; c_{B,A,(bp,BP3550),F,122}) = (0.9729, 0.027, \, 0.8832)$$

The functional reputation for taking blood pressure measurements with the Braun BP3550 can be calculated using the ratings for health data and the rule ratings. For this, the functional reputation part using ratings for health data needs to be computed. The functional reputation part based on rule ratings needs to be calculated separately.

The scope function is defined as:

|              | (bp,sphyg) | (bp,BP3550) | (bp,HEM650) |
|--------------|------------|-------------|-------------|
| **(bp,sphyg)**   | 1          | 0.3         | 0.3         |
| **(bp,BP3550)**  | 0.3        | 1           | 0.8         |
| **(bp,HEM650)**  | 0.3        | 0.8         | 1           |

The first forgetting function is represented by:

$$f(i,j) = \frac{1}{(1+j-i)^{\lambda}}$$

With λ=0.25

The second forgetting function is represented by:

$$g(i,j) = \lambda^{j-i}$$

With λ=0.9999

The functional reputation value for taking blood pressure measurements with the Braun BP3550 of Alice for Bob at time t = 122 is then calculated as follows:

$$RH_{B,A,(bp,BP3550),F,122} \quad = \quad SS((bp,BP3550),(bp,sphyg)) \cdot \begin{pmatrix} g(101,122) \cdot f(1,6) \cdot 0.2 \cdot 0.5 + \\ \vdots \\ g(108,122) \cdot f(6,6) \cdot 0.7759 \cdot 0.1) \end{pmatrix}$$

$$+ \quad SS((bp,BP3550),(bp,BP3550)) \cdot \begin{pmatrix} g(109,122) \cdot f(1,5) \cdot 0.6 \cdot 0.8 + \\ \vdots \\ g(122,122) \cdot f(5,5) \cdot 0.8832 \cdot 0.9729) \end{pmatrix}$$

$$= \quad 2.4179$$

$$SH_{B,A,(bp,BP3550),F,122} \quad = \quad SS((bp,BP3550),(bp,sphyg)) \cdot \begin{pmatrix} g(101,122) \cdot f(1,6) \cdot 0.2 \cdot 0.5 + \\ \vdots \\ g(108,122) \cdot f(6,6) \cdot 0.7759 \cdot 0.9) \end{pmatrix}$$

$$+ \quad SS((bp,BP3550),(bp,BP3550)) \cdot \begin{pmatrix} g(109,122) \cdot f(1,5) \cdot 0.6 \cdot 0.2 + \\ \vdots \\ g(122,122) \cdot f(5,5) \cdot 0.8832 \cdot 0.0271) \end{pmatrix}$$

$$= \quad 0.6982$$

The functional reputation part based on rule ratings computed as follows:

$$RR_{A,(bp,BP3550),F,122} \quad = \quad \sum_{i=1}^{|R|} c_{A,(bp,BP3550),F,t} \cdot r_{A,(bp,BP3550),F,t}$$

$$= \quad 0.1 \cdot 0.7$$

$$= \quad 0.07$$

$$SR_{A,(bp,BP3550),F,122} \quad = \quad \sum_{i=1}^{|R|} c_{A,(bp,BP3550),F,t} \cdot s_{A,(bp,BP3550),F,t}$$

$$= \quad 0.1 \cdot 0.3$$

$$= \quad 0.03$$

The reputation of Bob for Alice can be calculated by combining the reputation part based on ratings for health data and the reputation part based on rule ratings:

$$R_{B,A,(bp,BP3550),F,122} \quad = \quad RH_{B,A,(bp,BP3550),F,122} + \omega \cdot RR_{A,(bp,BP3550),F,122}$$

$$= \quad 2.4179 + 25 \cdot 0.07$$

$$= \quad 4.1679$$

$$S_{B,A,(bp,BP3550),F,122} = SH_{B,A,(bp,BP3550),F,122} + \omega \cdot SR_{A,(bp,BP3550),F,122}$$
$$= 0.6982 + 25 \cdot 0.03$$
$$= 1.4482$$

Blood pressure (part 2):

Ten years later, Alice is again diagnosed with high blood pressure. In the meantime, Dr. David has taken over Bob's doctor's practice. David is not sure whether he should trust the measurements provided by Alice. David has never given any ratings on the measurements provided by Alice. Because David trusts Bob for providing recommendations on doing measurements, David can trust Bob's ratings on Alice's measurements. Therefore, in David's eyes, Alice has a good reputation for measuring blood pressure with the BP3550 and a bad reputation for measuring blood pressure using a sphygmomanometer. Because the reputation is based on 10-year-old measurements it expresses more uncertainty than 10 years ago. Moreover, certainty is decreased because David is computing the reputation based on ratings provided by Bob. Also, Alice has bought a new blood pressure meter, the Omron HEM650 with advanced positioning sensors. Like the BP3550, the HEM650 also provides metadata on whether the arm was positioned on the right level. Alice has no ratings for measuring blood pressure using the HEM650. Therefore, the ratings for measuring blood pressure using a sphygmomanometer and using the BP3550 are used to calculate the reputation for using the HEM650. Because using the BP3550 is more similar to using the HEM650 than using the sphygmomanometer, the ratings for using the BP3550 are given more weight than the ratings for using the sphygmomanometer.

The local ratings given by Bob and Charlie in the past can be used by David to calculate Alice's reputation. However, the ratings need to be discounted by the recommendation reputation of David for Bob and Charlie. Throughout the scenario, it is assumed that Bob and Charlie have the following recommendation reputation (for all t):

$$R_{D,C,(bp,BP3550),R,t} = 11.875 \qquad S_{D,C,(bp,\ BP3550),R,t} = 0.625$$

$$R_{D,C,(bp,sphyg),R,t} = 11.25 \quad S_{D,C,(bp,sphyg),R,t} = 1.25$$

$$R_{D,B,(bp,sphyg),R,t} = 11.25 \quad S_{D,B,(bp,sphyg),R,t} = 1.25$$

The global ratings of David for Alice are the following:

$$(r_{D,A,(bp,sphyg),101},\ s_{D,A,(bp,sphyg),101},\ c_{D,A,(bp,sphyg),101}) = (0.5,\ 0.5,\ 0.1552)$$

$$(r_{D,A,(bp,sphyg),102},\ s_{D,A,(bp,sphyg),102},\ c_{D,A,(bp,sphyg),102}) = (0.5,\ 0.5,\ 0.1552)$$

$$(r_{D,A,(bp,sphyg),103},\ s_{D,A,(bp,sphyg),103},\ c_{D,A,(bp,sphyg),103}) = (0.5,\ 0.5,\ 0.1552)$$

$$(r_{D,A,(bp,sphyg),104},\ s_{D,A,(bp,sphyg),104},\ c_{D,A,(bp,sphyg),104}) = (0.5,\ 0.5,\ 0.1552)$$

$$\left(r_{D,A,(bp,sphyg),105},\ s_{D,A,(bp,sphyg),105},\ c_{D,A,(bp,sphyg),105}\right) = (0.5,\ 0.5,\ 0.1552)$$

$$\left(r_{D,A,(bp,sphyg),108},\ s_{D,A,(bp,sphyg),108},\ c_{D,A,(bp,sphyg),108}\right) = (0.1,\ 0.9,\ 0.7759)$$

$$\left(r_{D,A,(bp,BP3550),109},\ s_{D,A,(bp,BP3550),109},\ c_{D,A,(bp,BP3550),109}\right) = (0.8,\ 0.2,\ 0.4914)$$

$$\left(r_{D,A,(bp,BP3550),110},\ s_{D,A,(bp,BP3550),110},\ c_{D,A,(bp,BP3550),110}\right) = (0.8,\ 0.2,\ 0.4914)$$

$$\left(r_{D,A,(bp,BP3550),111},\ s_{D,A,(bp,BP3550),111},\ c_{D,A,(bp,BP3550),111}\right) = (0.8,\ 0.2,\ 0.4914)$$

$$\left(r_{D,A,(bp,BP3550),112},\ s_{D,A,(bp,BP3550),112},\ c_{D,A,(bp,BP3550),112}\right) = (0.8,\ 0.2,\ 0.4914)$$

The aggregation rating for the measurements taken by Alice and Bob at t = 122 can also be calculated by David:

$$\left(r_{D,A,(bp,BP3550),F,122}, s_{D,A,(bp,BP3550),F,122}, c_{D,A,(bp,BP3550),F,122}\right) = (0.9729,\ 0.0271,\ 0.8832)$$

"When Alice provides blood pressure measurements with the HEM650, David is provided with the reputation information about Alice."

The functional reputation at t = 3750 is calculated as follows:

$$
\begin{aligned}
R_{D,A,(bp,HEM650),F,3750} \quad = \quad & SS((bp,HEM650),(bp,sphyg)) \cdot \begin{pmatrix} g(101,3750)\cdot f(1,6)\cdot 0.1552\cdot 0.5 + \\ \vdots \\ g(108,3750)\cdot f(6,6)\cdot 0.7759\cdot 0.1 \end{pmatrix} \\
+ \quad & SS((bp,HEM650),(bp,BP3550)) \cdot \begin{pmatrix} g(109,3750)\cdot f(1,5)\cdot 0.4919\cdot 0.8 + \\ \vdots \\ g(122,3750)\cdot f(5,5)\cdot 0.8832\cdot 0.9729 \end{pmatrix} \\
= \quad & 1.2034
\end{aligned}
$$

$$S_{D,A,(bp,HEM\,650),F,3750} \;=\; SS((bp,HEM\,650),(bp,sphyg)) \cdot \begin{pmatrix} g(101,3750)\cdot f(1,6)\cdot 0.1552\cdot 0.5\,+ \\ \vdots \\ g(108,3750)\cdot f(6,6)\cdot 0.7759\cdot 0.9) \end{pmatrix}$$

$$+\; SS((bp,HEM\,650),(bp,BP3550)) \cdot \begin{pmatrix} g(109,3750)\cdot f(1,5)\cdot 0.4919\cdot 0.2\,+ \\ \vdots \\ g(122,3750)\cdot f(5,5)\cdot 0.8832\cdot 0.0271) \end{pmatrix}$$

$$=\; 0.3799$$

"Alice's reputation is quite good, but shows quite some uncertainty. The uncertainty is due to the old ratings and the different equipment. Therefore, David decides that he wants to redo a measurement. Because the measurements provided by Alice and David are similar, David gives Alice a good rating. Alice's reputation is increased and the certainty of this reputation is increased. From now on, David trusts Alice's measurements, as long as the metadata shows that the arm position is alright."

The rating provided by David is the following:

$$(r_{D,A,(bp,HEM650),F,3750},\; s_{D,A,(bp,HEM650),F,3750},\; c_{D,A,(bp,HEM650),F,3750}) = (1,0,1)$$

The reputation of Alice is now:

$$R_{D,A,(bp,HEM650),F,3750} = 2.2034 \qquad S_{D,A,(bp,HEM650),F,3750} = 0.3799$$

End example 1

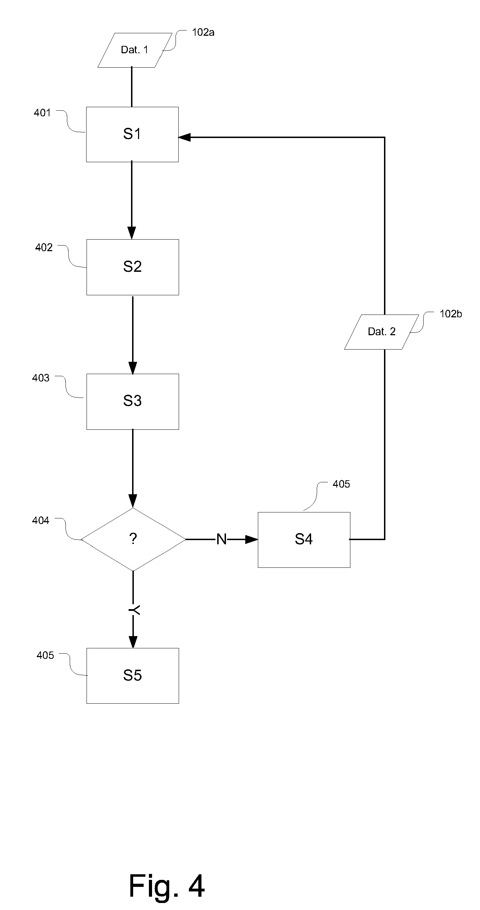

Figure 4 shows a flowchart of a method according to the present invention for providing a measure of reliability of a first set on health data 102a on a patient provided by a data provider. In a first step (S1) 401 the health data or the data provider are assigned with at least one rating element, and the assigned rating element is used (S2) 402 as input data in determining (S3) 403 a first reputation measure, the first reputation measure indicating the reliability of the data provider. The first reputation measure is compared 404 with a pre-defined reputation threshold measure, the reputation threshold measure being a measure of a pre-set reliability level set by the healthcare provider. In one embodiment, if the reputation measure is below the reputation threshold measure (S4) 405, a second set of health data is created resulting in a second data set 102b and steps S1-S3 are repeated. Otherwise, the first set of health data 102a is considered to be reliable (S5) 405.

Certain specific details of the disclosed embodiment are set forth for purposes of explanation rather than limitation, so as to provide a clear and thorough understanding of the present invention. However, it should be understood by those skilled in this art, that the present invention might be practiced in other embodiments that do not conform exactly to the details set forth herein, without departing significantly from the spirit and scope of this disclosure. Further, in this context, and for the purposes of brevity and clarity, detailed descriptions of well-known apparatuses, circuits and methodologies have been omitted so as to avoid unnecessary detail and possible confusion.

Reference signs are included in the claims, however the inclusion of the reference signs is only for clarity reasons and should not be construed as limiting the scope of the claims.

**Claims**

1. A reputation system for providing a measure of reliability on a first set of health data (102) on a patient (103) provided by a data provider, the system comprising:

a) an assigner (105) for assigning the first set of health data or the data provider with at least one first rating element,

b) a reputation-indicator (106) for using the at least one first rating element as input data in determining a first reputation measure indicating the reliability of the data provider, and

c) a comparer (107) for comparing the determined first reputation measure with a pre-defined reputation threshold measure, the reputation threshold measure being a measure of a pre-set reliability level of the data provider.

2. A reputation system according to claim 1, wherein the assigner comprises a rule engine (202) adapted to determine ratings for the data provider associated with certificates of the data provider.

3. A reputation system according to claim 1, wherein the assigner comprises an aggregation engine (207) adapted to determine ratings for the health data based on comparing two or more health data sets on the same patient provided by different data providers.

4. A reputation system according to claim 3, wherein the aggregation engine (207) determines the ratings for the health data by calculating the consistency between the two or more health data sets.

5. A reputation system according to claim 1, wherein the assigner (105) comprises a healthcare provider or a wellness provider which assigns by a manual operation the health data with the at least one rating element.

6. A reputation system according to claim 1, wherein the rating means comprises a metadata source for incorporating metadata (104) to the health data, the metadata being implemented as additional data source in assigning the health data with at least one rating element.

7. A reputation system according to claim 1, wherein the data provider is the patient or a wellness provider.

8. A reputation system according to claim 1, wherein the data provider is a healthcare provider (209).

9. A reputation system according to claim 1, wherein the first set of health data (102) as well as the accompanying metadata (104) is obtained from an external database (109) or system over a communication channel (111).

10. A reputation system according to claim 1, wherein the first reputation measure related to health data (102) is sent over a communication channel (111) to a remote system.

11. A reputation system according to claim 1, wherein the rating relating to a first set of health data (102) is sent over a communication channel (111) to a remote system.

12. A method of providing a measure of reliability on a first set of health data on a patient provided by a data provider, comprising:

a) assigning (401) the first set of health data or the data provider with at least one rating element,

b) using the assigned rating element (402) as input data (403) in determining a first reputation measure indicating the reliability of the data provider, and

c) comparing the determined first reputation measure with a pre-defined reputation threshold measure (404), the reputation threshold measure being a measure of a pre-set reliability level of the data provider.

13. A computer program product for instructing a processing unit to execute the method step of claim 12 when the product is run on a computer.

**Fig. 1**

Cert. — 201

R.-eng — 202

R.-rat. — 203

207 — Agg. Eng.

206 — Agg. Rat.

204/106 — Rep.-eng

205 — Loc./Glob.rat.

208 — H.-Dat.

211 — Rep.

209 — H-C-P

210 — M.-Dat.

213 — Dec.

## Fig. 2

H.C.P — 301

PHR sys. — 302

$req(y, sc, t)$

$hd_{z,y,sc,t}, md_{z,y,sc,t}, (R_{x,z,sc,F,t}, S_{x,z,sc,F,t})$

$(r_{x,z,sc,F,t}, s_{x,z,sc,F,t}, c_{x,z,sc,F,t})$

## Fig. 3

Fig. 4

**European Patent Office**

**EUROPEAN SEARCH REPORT**

Application Number

EP 07 10 9798

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 02/061549 A (POTGIETER HERMANUS JOHANNES [ZA]) 8 August 2002 (2002-08-08) * page 2, line 1 - page 5, line 11 * * page 7, line 10 - page 12, line 15 * ----- | 1-13 | INV. G06F19/00 |
| X | US 7 031 952 B1 (HEUMANN MICHAEL CARL [US] ET AL) 18 April 2006 (2006-04-18) * column 2, line 29 - column 3, line 30 * * column 3, line 34 - line 60 * * column 10, line 31 - column 11, line 44 * ----- | 1-13 | |
| A | US 2002/004758 A1 (TAKECHI MINEKI [JP]) 10 January 2002 (2002-01-10) * paragraph [0006] - paragraph [0013] * * paragraph [0024] - paragraph [0031] * * paragraph [0033] - paragraph [0048] * * paragraph [0050] - paragraph [0065] * ----- | 1-13 | |
| A | EP 1 233 354 A (HITACHI LTD [JP]) 21 August 2002 (2002-08-21) * column 3, line 3 - column 6, line 34 * * column 7, line 15 - column 13, line 48 * ----- | 1-13 | TECHNICAL FIELDS SEARCHED (IPC) G06F |
| A | EP 1 566 758 A (AT & T CORP [US]) 24 August 2005 (2005-08-24) * column 5, line 24 - column 6, line 26 * * column 6, line 50 - column 12, line 47 * ----- | 1-13 | |
| A | US 2003/101166 A1 (UCHINO KANJI [JP] ET AL) 29 May 2003 (2003-05-29) * paragraph [0010] - paragraph [0024] * * paragraph [0043] - paragraph [0063] * * paragraph [0073] - paragraph [0097] * ----- -/-- | 1-13 | |

The present search report has been drawn up for all claims

| Place of search | Date of completion of the search | Examiner |
|---|---|---|
| Munich | 4 October 2007 | Barba, Michelangelo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent
Office

**EUROPEAN SEARCH REPORT**

Application Number

EP 07 10 9798

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2004/031982 A (TENIX INVEST PTY LTD [AU]; SYKES MICHAEL JOHN [AU]; WEINSTEIN DANIEL S) 15 April 2004 (2004-04-15)<br>* page 2, line 11 - page 3, line 9 *<br>* page 6, line 11 - line 27 *<br>* page 7, line 7 - page 10, line 27 *<br>* page 12, line 7 - page 13, line 4 *<br>----- | 1-13 | |
| A | CHAUDHURI S ET AL: "An Overview of Data Warehousing and OLAP Technology"<br>SIGMOD RECORD, SIGMOD, NEW YORK, NY, US, vol. 26, no. 1, March 1997 (1997-03), pages 65-74, XP002193792<br>ISSN: 0163-5808<br>* page 67, left-hand column, line 29 - right-hand column, line 17 *<br>* page 73, left-hand column, line 15 - right-hand column, line 18 *<br>----- | 1-13 | |
| A | LEITHEISER R L: "Data quality in health care data warehouse environments"<br>SYSTEM SCIENCES, 2001. PROCEEDINGS OF THE 34TH ANNUAL HAWAII INTERNATIONAL CONFERENCE ON JANUARY 3-6, 2001, PISCATAWAY, NJ, USA,IEEE,<br>3 January 2001 (2001-01-03), pages 2114-2123, XP010549832<br>ISBN: 0-7695-0981-9<br>* page 2114, left-hand column, line 15 - right-hand column, line 10 *<br>* page 2114, right-hand column, line 12 - page 2115, right-hand column, line 27 *<br>* page 2116, left-hand column, line 14 - page 2117, right-hand column, line 52 *<br>* page 2118, left-hand column, line 11 - page 2119, right-hand column, line 50 *<br>* page 2120, left-hand column, line 11 - line 36 *<br>-----<br><br>-/-- | 1-13 | TECHNICAL FIELDS SEARCHED (IPC) |

The present search report has been drawn up for all claims

| Place of search | Date of completion of the search | Examiner |
|---|---|---|
| Munich | 4 October 2007 | Barba, Michelangelo |

EPO FORM 1503 03.82 (P04C01)

| | **European Patent Office** | **EUROPEAN SEARCH REPORT** | | **Application Number** EP 07 10 9798 |
|---|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZACHARIA G ET AL: "Trust management through reputation mechanisms" APPLIED ARTIFICIAL INTELLIGENCE TAYLOR & FRANCIS UK, vol. 14, no. 9, October 2000 (2000-10), pages 881-907, XP002453769 ISSN: 0883-9514 * the whole document * | 1-13 | |
| A | WANG R Y: "A product perspective on total data quality management" COMMUNICATIONS OF THE ACM ACM USA, vol. 41, no. 2, February 1998 (1998-02), pages 58-65, XP002453770 ISSN: 0001-0782 * page 60, left-hand column, line 3 - page 62, left-hand column, line 21 * * page 62, right-hand column, line 20 - page 64, right-hand column, line 28 * | 1-13 | |
| A | JADAD A R ET AL: "Rating health information on the Internet: navigating to knowledge or to Babel?" JAMA : THE JOURNAL OF THE AMERICAN MEDICAL ASSOCIATION 25 FEB 1998, vol. 279, no. 8, 25 February 1998 (1998-02-25), pages 611-614, XP002453771 ISSN: 0098-7484 * the whole document * | 1-13 | TECHNICAL FIELDS SEARCHED (IPC) |

The present search report has been drawn up for all claims

| Place of search | Date of completion of the search | Examiner |
|---|---|---|
| Munich | 4 October 2007 | Barba, Michelangelo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                EP 07 10 9798

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2007

| Patent document cited in search report | | Publication date | Patent family member(s) | | Publication date |
|---|---|---|---|---|---|
| WO 02061549 | A | 08-08-2002 | NONE | | |
| US 7031952 | B1 | 18-04-2006 | NONE | | |
| US 2002004758 | A1 | 10-01-2002 | JP | 2002024702 A | 25-01-2002 |
| EP 1233354 | A | 21-08-2002 | CN | 1371072 A | 25-09-2002 |
| | | | JP | 2002245153 A | 30-08-2002 |
| | | | US | 2002120870 A1 | 29-08-2002 |
| EP 1566758 | A | 24-08-2005 | CA | 2496851 A1 | 18-08-2005 |
| | | | US | 2005182739 A1 | 18-08-2005 |
| US 2003101166 | A1 | 29-05-2003 | AU | 2002343775 A1 | 10-06-2003 |
| | | | CA | 2460538 A1 | 05-06-2003 |
| | | | CN | 1559044 A | 29-12-2004 |
| | | | EP | 1450268 A1 | 25-08-2004 |
| | | | WO | 03046764 A1 | 05-06-2003 |
| | | | TW | 252987 B | 11-04-2006 |
| WO 2004031982 | A | 15-04-2004 | CA | 2501205 A1 | 15-04-2004 |
| | | | EP | 1556797 A1 | 27-07-2005 |
| | | | US | 2005066240 A1 | 24-03-2005 |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. HANDLER ; R. HOLTMEIER ; J. METZGER ; M. OVERHAGE ; S. TAYLOR ; C. UNDERWOORD.** *HIMSS electronic health record definitional model version 1.1.,* 2003

- **A. JOSANG ; R. ISMAIL.** The Beta Reputation System. *In Proc. 15th Bled Conf. Electronic Commerce,* 2002